# FASCICULE DE BREVET EUROPEEN

(11) **EP 0 919 255 B1**
(45) Date de publication et mention de la délivrance du brevet: **26.05.2004**
(21) Numéro de dépôt: 98402943.9
(22) Date de dépôt: 25.11.1998
(51) Int. Cl.: A61N 1/365

(54) **Dispositif médical implantable actif, notamment stimulateur cardiaque, asservi à au moins un paramètre physiologique**
Implantierbare aktive medizinische Einrichtung, insbesondere Herzstimulator, gesteuert von wenigstens einem physiologischen Parameter
Implantable active medical device, especially pacemaker, responsive to a least one physiological parameter

(30) Priorité: 25.11.1997 FR 9714796
(43) Date de publication de la demande: 02.06.1999
(73) Titulaire: ELA MEDICAL (Société anonyme), 92541 Montrouge (FR)
(72) Inventeur: Bonnet, Jean-Luc, 92120 Montrouge (FR)
(74) Mandataire: Dupuis-Latour, Dominique

(56) Documents cités:
- EP-A- 0 702 980
- EP-A- 0 750 920
- WO-A-96/16695
- US-A- 4 945 909

## Description

La présente invention concerne les "dispositifs médicaux implantables actifs" tels que définis par la directive 90/385/CEE du 20 juin 1990 du Conseil des communautés européennes, notamment les stimulateurs cardiaques ou défibrillateurs dont le fonctionnement est asservi à un paramètre recueilli à l'aide d'un capteur.

A cet égard, bien que dans la suite de la description on fasse principalement référence au cas d'un stimulateur cardiaque, l'invention est applicable de façon générale à une très grande variété de dispositifs électroniques.

La demande de brevet EP 0 750 920 décrit l'utilisation de deux capteurs, un capteur d'activité et un capteur d'effort, et propose notamment une méthode pour sélectionner le capteur le plus rapide en début et en fin d'effort et le capteur physiologique durant l'effort. Un contrôle croisé a lieu dans la mesure où un capteur déterminant seul un effort, non confirmé par le second, ne pourra être pris en compte qu'un temps prédéterminé (typiquement 15 secondes pour le capteur d'activité et 2 minutes pour le capteur d'effort). Durant un effort confirmé par les deux capteurs l'asservissement aura lieu uniquement sur le capteur d'effort (c'est-à-dire le capteur pris en compte préférentiellement pendant l'effort) sans contrôle par le capteur d'activité. Selon ce principe de base de l'asservissement de la demande de brevet EP 0 750 920 (identique également au stimulateur cardiaque "Talent 213" de la société ELA Médical) un intervalle d'échappement est calculé pour le capteur d'activité toutes les 1,5625 secondes ce qui correspond à une nouvelle information du capteur. Tous les quatre cycles cardiaques celui-ci est moyenné sur les quatre derniers intervalles d'échappement. Un intervalle d'échappement est calculé pour le capteur d'effort tous les cycles respiratoires. Tous les quatre cycles cardiaques, celui-ci est moyenné sur les huit derniers intervalles d'échappement. Les valeurs d'intervalle d'échappement du capteur d'effort et du capteur d'activité permettent de déterminer les états des capteurs.

Les deux capteurs calculent indépendamment un intervalle d'échappement. A partir de l'évolution des intervalles d'échappement, on définit pour chaque capteur un état. Il existe trois états :
- 1er état - repos :: le capteur détermine un état de repos du patient
- 2ème état - effort :: le capteur détermine un état d'effort "croissant" du patient
- 3ème état - récupération :: le capteur détermine une fin d'effort ce qui est un état intermédiaire entre l'état d'effort et l'état de repos.

Étant donné qu'il y a deux capteurs et trois états, il existe neuf combinaisons d'états. Pour chacune des combinaisons, le stimulateur adopte un comportement différent. Chaque combinaison détermine une valeur de l'intervalle d'échappement de consigne, c'est-à-dire de l'intervalle d'échappement vers lequel doit tendre la période d'asservissement.

Mais l'asservissement à double capteur, tel que décrit précédemment et objet de la demande européenne EP 0 750 920, est principalement basé sur l'information du capteur d'effort. Or, il peut arriver que l'intervalle d'échappement défini par ce capteur ne soit pas en concordance avec le niveau d'activité du patient, en cas des mouvements importants par exemple. Dans ce cas, l'intervalle d'échappement de consigne et la période d'asservissement peuvent avoir des valeurs ne correspondant pas aux besoins du patient, ou trop basses, ou trop élevées.

L'invention a pour objet de résoudre ce problème et de définir un contrôle de l'intervalle d'échappement de consigne dans le cas où il est égal à l'intervalle d'échappement défini par le capteur d'effort.

Ce problème est résolu par un dispositif médical implantable actif susceptible d'un fonctionnement asservi par pilotage d'au moins une fonction du dispositif par au moins un paramètre physiologique. Ce dispositif, du type général décrit par le EP-A-0 750 920 précité, comporte au moins un capteur d'effort, mesurant un paramètre à prépondérance physiologique et délivrant un signal fonction de l'effort développé par un patient porteur du dispositif, au moins un capteur d'activité mesurant un paramètre à prépondérance physique, des moyens pour analyser périodiquement la séquence relative des changements d'états successifs desdits capteurs en fonction de critères prédéterminés intrinsèques au dispositif, des moyens pour déterminer un intervalle d'échappement consigne pilotant le fonctionnement asservi du dispositif à partir des états analysés des capteurs, et des moyens pour limiter l'intervalle d'échappement de consigne à des valeurs prédéfinies. Selon l'invention, les limitations de l'intervalle d'échappement de consigne étant fonction de l'intervalle d'échappement défini par le capteur d'activité.

Selon une autre forme de réalisation avantageuse les limitations de l'intervalle d'échappement de consigne correspondent à la déduction d'un pourcentage constant prédéfini de l'intervalle d'échappement défini par le capteur d'activité.

De préférence, on applique deux limitations, une limite inférieure et une limite supérieure, à l'intervalle d'échappement de consigne.

De plus, d'une façon avantageuse, on redéfinit les deux limites inférieure et supérieure chaque fois que les moyens d'analyse des états des capteurs déterminent que l'intervalle d'échappement défini par le capteur d'activité est modifié.

Selon une autre forme de réalisation préférée, les moyens pour limiter l'intervalle d'échappement de consigne n'appliquent la limitation de l'intervalle d'échappement de consigne que dans le cas où le capteur d'effort est dans un état différent du repos.

De préférence, les moyens pour limiter l'intervalle d'échappement de consigne limitent la fréquence d'asservissement dans le cas où le capteur d'effort détecte un effort non détecté par le capteur d'activité.

D'autres caractéristiques et avantages de l'invention apparaîtront à la lecture de la description détaillée ci-dessous, faite en référence aux dessins annexés.
- La figure 1: est un organigramme du calcul de l'intervalle d'échappement de consigne tel que connu dans l'état de l'art.
- La figure 2: est un organigramme général du fonctionnement de l'invention.
- La figure 3: illustre l'évolution de la limitation en fonction de l'intervalle d'échappement défini par le capteur d'activité.
- La figure 4: montre la limitation de l'intervalle d'échappement de consigne.
- La figure 5: montre l'effet de la limitation par le capteur d'activité par échelons dans le cas normal.
- La figure 6: montre l'effet de la limitation par le capteur d'activité par échelons.
- La figure 7: montre l'effet de la limitation par le capteur d'activité.

Dans la description détaillée qui va suivre, on prendra l'exemple d'un stimulateur cardiaque asservi à un paramètre physiologique. Cette application n'est cependant pas limitative, et les enseignements de l'invention sont directement applicables à d'autres types de dispositifs médicaux implantables actifs.

De même, bien que l'on fera référence à deux capteurs seulement, on peut prévoir dans une version plus élaborée un nombre supérieur de capteurs, multiplexés ou combinés entre eux, permettant d'asservir le fonctionnement du dispositif à une pluralité de paramètres. Les types de capteurs décrits ici (à savoir un capteur de ventilation-minute pour le capteur d'effort MV et un capteur d'accélération pour le capteur d'activité G) ne sont également pas limitatifs, et d'autres types de capteurs peuvent être envisagés.

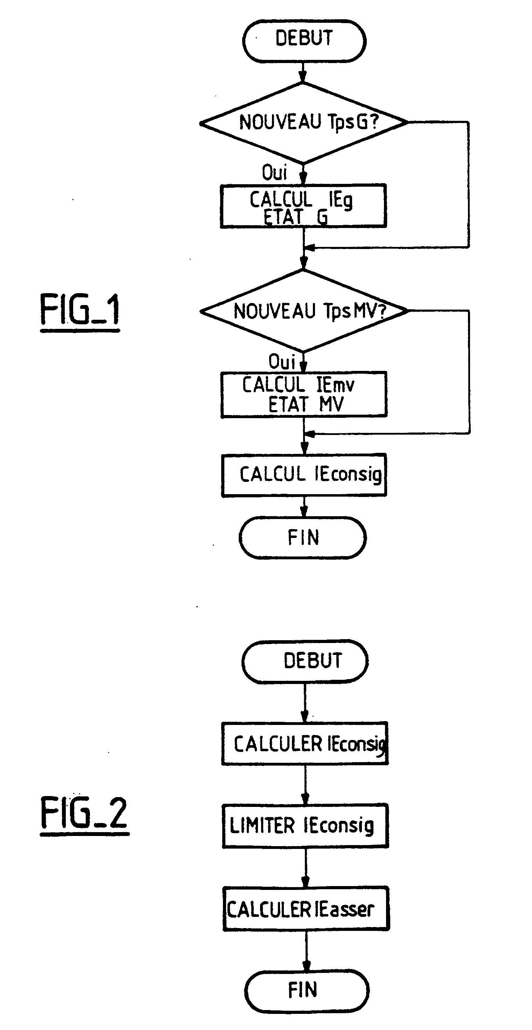

La Fig. 1 montre un organigramme du calcul de l'intervalle d'échappement consigne IEconsig tel que connu de la demande de brevet EP 0 750 920. Dans ce cycle de base, qui est mis en oeuvre systématiquement, par exemple tous les quatre cycles cardiaques, on examine d'abord l'état du capteur d'activité G et l'on teste s'il y a eu ou non une modification de ce capteur. Dans l'affirmative on calcule l'intervalle d'échappement IEg du capteur d'activité G, on détermine l'état du capteur G et on procède à l'analyse du capteur d'effort MV.

Dans la négative, on procède directement à l'analyse de l'état du capteur d'effort MV. S'il y a eu une modification de l'état du capteur MV, on calcule l'intervalle d'échappement IEmv du capteur MV et on détermine le nouvel état de ce capteur.

La manière de déterminer l'état des capteurs est décrite de façon plus détaillée dans la demande de brevet EP 0 750 920.

Une fois les deux capteurs testés, on calcule, qu'il y ait eu modification ou non des états des capteurs, l'intervalle d'échappement consigne IEconsig. Le tableau 1 montre ce calcul.

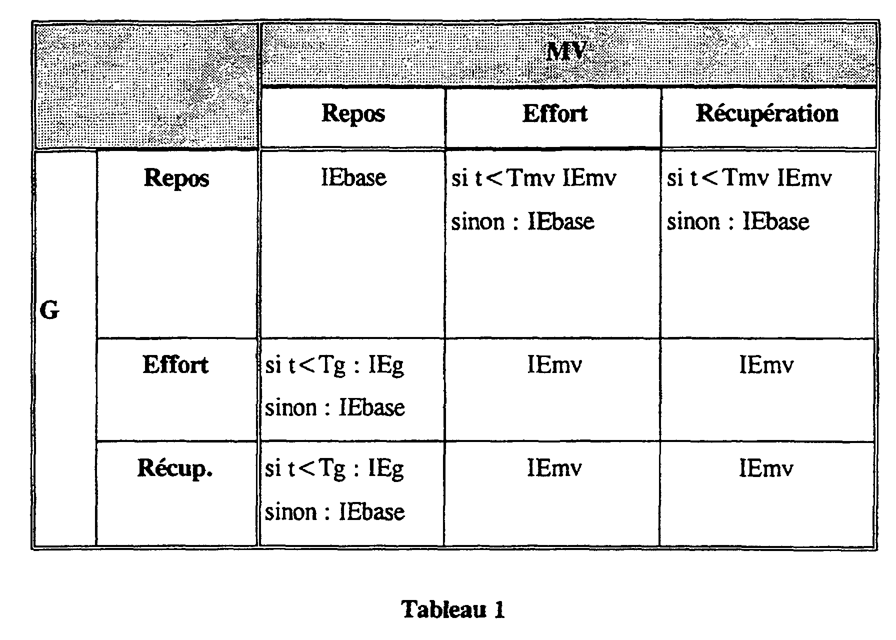

Comme cela est montré dans le tableau 1, l'intervalle d'échappement de consigne IEconsig ne peut prendre que trois valeurs : IEbase (l'intervalle d'échappement correspondant à la fréquence de base programmée), IEg (l'intervalle d'échappement du capteur G) ou IEmv (l'intervalle d'échappement du capteur MV).
- Si les capteurs G et MV sont à l'état de repos, l'intervalle d'échappement consigne IEconsig est égal à l'intervalle d'échappement IEbase puisqu'on considère qu'il s'agit d'un repos confirmé par les deux capteurs.
- Si les deux capteurs sont à l'état d'effort ou l'état de récupération, l'intervalle d'échappement de consigne IEconsig est égal à l'intervalle d'échappement IEmv du capteur MV puisqu'on considère qu'il s'agit d'un effort confirmé par les deux capteurs.
- Si le capteur G est à l'état de repos et le capteur MV est à un état différent de repos, l'effort n'est pas confirmé et on définit que l'intervalle d'échappement de consigne IEconsig est égal à l'intervalle d'échappement IEmv du capteur MV pendant un temps limité Tmv. Au delà de ce temps, l'intervalle d'échappement consigne IEconsig est égal à IEbase.
- Si le capteur MV est à l'état de repos et le capteur G est à un état différent de repos, l'effort n'est pas confirmé et on définit que l'intervalle d'échappement de consigne IEconsig est égal à l'intervalle d'échappement IEg du capteur d'activité G pendant un temps limité Tg. Au delà de ce temps, l'intervalle d'échappement consigne IEconsig est égal à IEbase.

Le capteur d'activité G est typiquement un capteur d'accélération. Au cours d'un effort important, il répond de façon appropriée. Cependant le capteur d'activité G n'est pas considéré comme physiologique, car il ne délivre pas de signal proportionnel au niveau d'effort. Le capteur d'effort, le capteur MV, délivre en revanche un signal proportionnel au niveau de l'effort. Cependant, certains artefacts externes tels que des mouvements rythmiques dans la bande passante de la ventilation peuvent leurrer le capteur, qui indiquera une fréquence de stimulation exagérée par rapport au niveau d'effort.

Pour remédier à ce problème, on définit des limites inférieure et supérieure de l'intervalle d'échappement de consigne IEconsig en fonction de l'intervalle d'échappement IEg du capteur d'activité G. En effet, les bandes passantes de ces deux capteurs MV et G ne se recouvrent pas ; les artefacts de l'un sont filtrés par l'autre. Puisque l'intervalle d'échappement consigne IEconsig est la valeur vers laquelle l'intervalle d'échappement asservi IEasser (la période de stimulation déterminée par la fonction d'asservissement) doit tendre, ce dernier sera contrôlé par l'intervalle d'échappement IEg du capteur d'activité G.

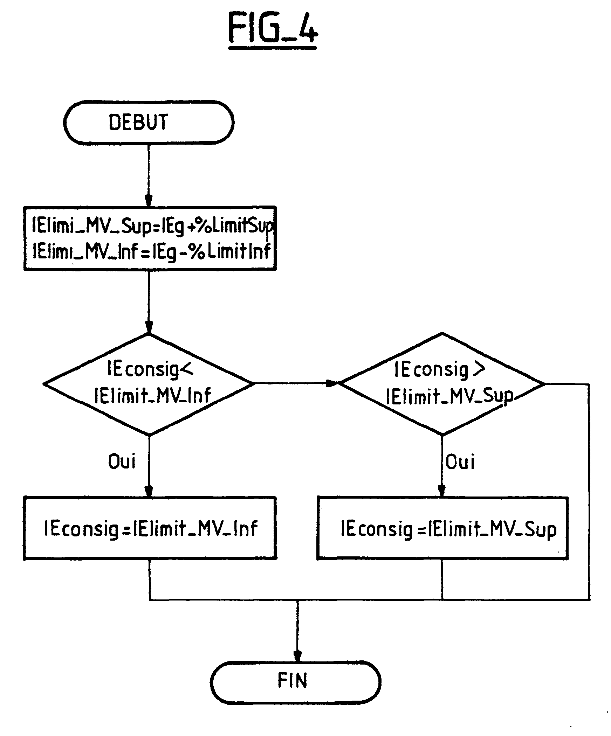

La figure 2 montre un organigramme général du fonctionnement de l'invention. La première étape est le calcul de l'intervalle d'échappement de consigne IEconsig tel que c'est connu de la demande de brevet EP 0 750 920 (voir figure 1). Après on calcule les limites de l'intervalle d'échappement de consigne IEconsig comme cela sera décrit plus en détail ci-dessous par rapport à la figure 4 et l'intervalle d'échappement asservi IEasser ou la fréquence asservie Fasser. Le calcul de la fréquence asservie est décrit d'une façon détaillée dans la demande de brevet EP 0 750 920 et ne sera pas décrit ici.

A partir de la valeur de l'intervalle d'échappement IEg du capteur G, on définit une valeur limite pour l'intervalle d'échappement IEmv du capteur MV. Il y a plusieurs façons de limiter l'intervalle d'échappement IEmv. Soit on définit une limite absolue en déduisant une période constante prédéfinie de l'intervalle d'échappement IEg (IEg - X ms). Soit on définit une limite relative en déduisant un pourcentage constant prédéfini de l'intervalle d'échappement IEg (IEg - %IEg). La limite de l'intervalle d'échappement IEmv peut aussi être définie par une fonction de l'intervalle d'échappement IEg du capteur d'activité (f(IEg)).

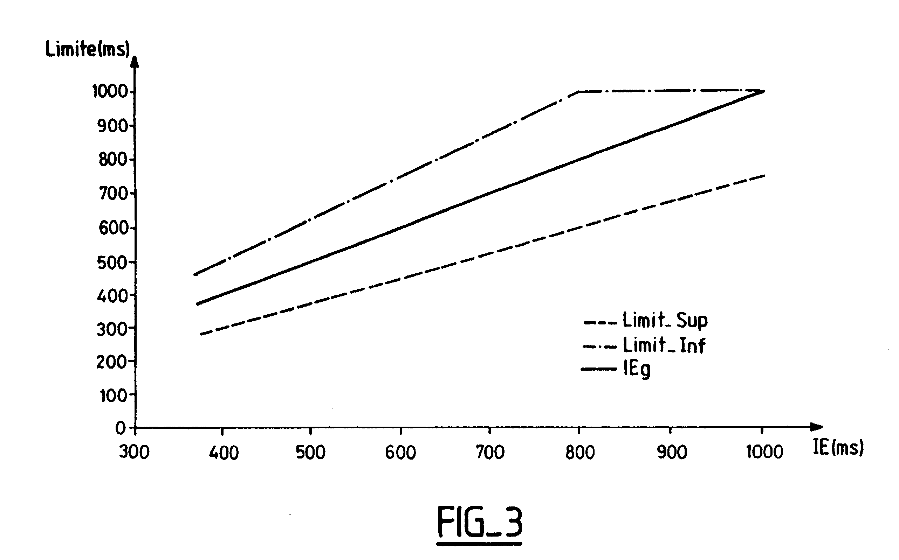

Selon une forme de réalisation préférée de l'invention, on définit deux limites, l'une pour la limitation inférieure de l'intervalle d'échappement de consigne IEconsig (IElimit_MV_Inf) et l'autre pour la limitation supérieure de cet intervalle (IElimit_MV_Sup). La figure 3 montre le cas d'application d'une limite relative. Les limites inférieure et supérieure sont un pourcentage de l'intervalle d'échappement IEg du capteur d'activité. La limite inférieure de l'intervalle d'échappement IEmv (IElimit_MV_Inf = IEg - 25 %IEg ; en trait mixte sur la figure 3) et la limite supérieure de cette intervalle (IElimit_MV_Sup = IEg + 25 %IEg ; en tiretés sur la figure 3) sont proportionnelles à l'intervalle d'échappement IEg du capteur d'activité (en trait plein).

A chaque fois qu'on a constaté une modification de l'intervalle d'échappement IEg du capteur G (voir figure 1), on redéfinit les deux limites IElimit_MV_Inf et IElimit_MV_sup de l'intervalle d'échappement IEmv du capteur d'effort. Dès que l'intervalle d'échappement de consigne IEconsig - calculé selon le tableau 1 - dépasse l'une de ces limites, il est limité à cette valeur limite.

La figure 4 montre la comparaison de la valeur de l'intervalle d'échappement de consigne IEconsig avec les limites inférieures et supérieures et la limitation conséquente de IEconsig.

Lorsque le capteur d'effort détermine seul un effort, la fréquence d'asservissement peut donc augmenter durant un temps prédéfini Tmv jusqu'à la valeur limite. Au delà de ce temps, si la confirmation de l'effort par le capteur d'activité G n'est toujours pas arrivée, la fréquence d'asservissement est diminuée jusqu'à la fréquence de base correspondant à IEbase. Par conséquent, la fréquence d'asservissement sera limitée par la limite inférieure IElimit_MV_Inf. Si, par exemple, l'intervalle d'échappement IEg du capteur d'effort est égal à l'intervalle d'échappement de base programmé IEbase ayant une valeur de 1000 ms et la limite relative prédéfinie est 25% de IEg on obtient une fréquence d'asservissement maximale de 60 000/(1000 ms - 250 ms) = 80 cpm. La fréquence d'asservissement est généralement définie comme la fréquence de stimulation déterminée par la fonction d'asservissement (= 60000/période d'asservissement).

Au cours d'un effort la période d'asservissement sera limitée à chaque instant en fonction du capteur d'activité. Par exemple, pour un intervalle d'échappement IEg du capteur d'activité égal à 600 ms (100 cpm) et une limite de 25%, la valeur minimale de IEmv sera 450 ms (133 cpm).

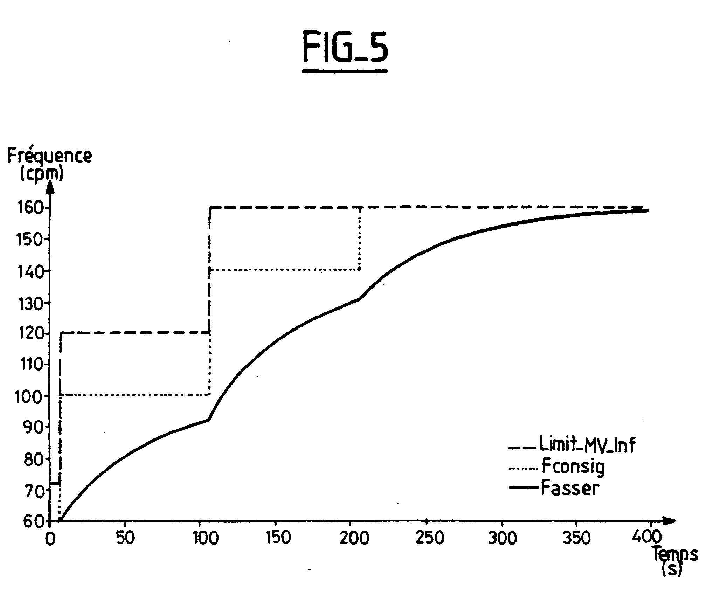

La figure 5 montre le cas normal dans lequel la limite inférieure de l'intervalle d'échappement IE (IElimit_MV_Inf ; en tirets) est inférieure à l'intervalle d'échappement de consigne IEconsig. Sur la figure 5 la fréquence de consigne, montrée en pointillés, est donc inférieure à la fréquence limite (en tirets) et, par conséquent la fréquence asservie Fasser (en trait continu) suit la fréquence de consigne Fconsig.

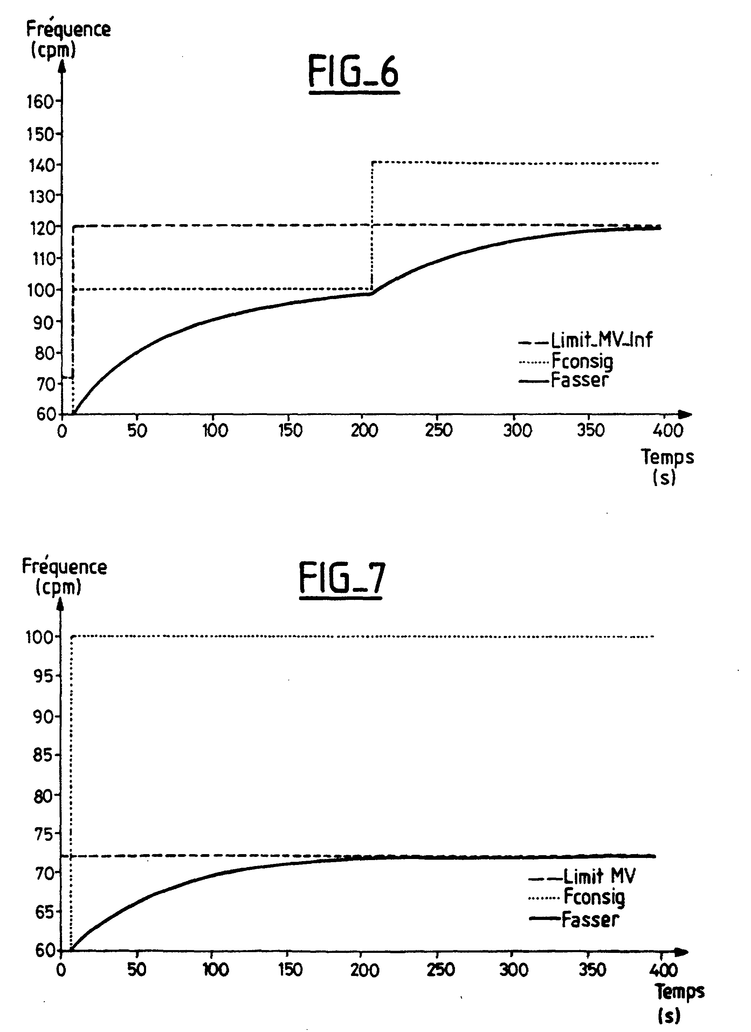

Dans le cas de la figure 6 la fréquence de consigne Fconsig (en pointillés) est supérieure à la fréquence limite définie Flimit_MV_Inf (en tirets) lors du deuxième escalier. La fréquence asservie Fasser (en trait continu) suivra donc la fréquence limite.

Dans une autre variante de réalisation préférée on définit que la limitation de l'intervalle d'échappement consigne IEconsig n'est appliquée que dans les cas où les capteurs d'effort MV et d'activité G sont tous les deux dans un état différent du repos. Ainsi la limitation ne s'appliquerait pas pour un effort déterminé par le capteur d'effort MV seul et non confirmé par le capteur d'activité G. Cela peut être le cas d'un stress, par exemple.

De façon générale ce principe peut être étendu à tout capteur. On limite donc un premier capteur par un deuxième capteur (ou une combinaison limitée de plusieurs capteurs), le premier capteur étant de préférence un capteur physiologique et le deuxième capteur étant un capteur d'activité (un capteur d'accélération ou de pression).

Selon une forme de réalisation préférée de l'invention un contrôle croisé de l'effort est prévu. Si le capteur d'effort MV détecte un effort, et que celui-ci n'est pas détecté par le capteur d'activité G, la fréquence d'asservissement sera limitée. La figure 7 décrit un tel exemple. On peut donc supprimer le temps d'asservissement sur un capteur seul, avant confirmation par le second.

## Revendications

1. Un dispositif médical implantable actif susceptible d'un fonctionnement asservi par pilotage d'au moins une fonction du dispositif par au moins un paramètre physiologique, ce dispositif comportant :
a) au moins un capteur d'effort (MV), mesurant un paramètre à prépondérance physiologique et délivrant un signal fonction de l'effort développé par un patient porteur du dispositif,
b) au moins un capteur d'activité (G) mesurant un paramètre à prépondérance physique, et
c) des moyens pour analyser périodiquement la séquence relative des changements d'états successifs desdits capteurs en fonction de critères prédéterminés intrinsèques au dispositif,
d) des moyens pour déterminer un intervalle d'échappement de consigne (IEconsig) pilotant le fonctionnement asservi du dispositif à partir des états analysés des capteurs, et
e) des moyens pour limiter l'intervalle d'échappement de consigne (IEconsig) à des valeurs prédéfinies,
**caractérisé en ce que** les limitations de l'intervalle d'échappement de consigne (IEconsig) sont fonction de l'intervalle d'échappement défini par le capteur d'activité (IEg).

2. Dispositif selon l'une des revendications précédentes, **caractérisé en ce que** les limitations de l'intervalle d'échappement de consigne (IEconsig) correspondent à la déduction d'un pourcentage constant prédéfini de l'intervalle d'échappement défini par le capteur d'activité (IEg).

3. Dispositif selon l'une des revendications précédentes, **caractérisé en ce qu'**on applique deux limitations, une limite inférieure (IElimit_MV_Inf) et une limite supérieure (IElimit_MV_Sup), à l'intervalle d'échappement de consigne (IEconsig).

4. Dispositif selon la revendication 3, **caractérisé en ce qu'**on redéfinit les deux limites inférieure et supérieure (IElimit_MV_Inf ; IElimit_MV_Sup) à chaque fois que les moyens d'analyse des états des capteurs déterminent que l'intervalle d'échappement défini par le capteur d'activité (IEg) est modifié.

5. Dispositif selon l'une des revendications précédentes, **caractérisé en ce que** les moyens pour limiter l'intervalle d'échappement de consigne (IEconsig) n'appliquent la limitation de l'intervalle d'échappement de consigne (IEconsig) que dans le cas où le capteur d'effort (MV) est dans un état différent du repos.

6. Dispositif selon l'une des revendications précédentes, **caractérisé en ce que** les moyens pour limiter l'intervalle d'échappement de consigne (IEconsig) limitent la fréquence d'asservissement dans le cas où le capteur d'effort (MV) détecte un effort non détecté par le capteur d'activité (G).

## Patentansprüche

1. Aktive implantierbare medizinische Vorrichtung, geeignet für einen geregelten Betrieb durch Steuerung mindestens einer Funktion der Vorrichtung durch mindestens einen physiologischen Parameter, wobei die Vorrichtung aufweist:
a) mindestens einen Sensor der Belastung (MV), welcher einen überwiegend physiologischen Parameter misst und ein Signal liefert, das von der Belastung eines Patienten abhängig ist, welcher die Vorrichtung trägt,
b) mindestens einen Sensor der Aktivität (G), der einen überwiegend physischen Parameter misst, und
c) Mittel, um periodisch die relative Sequenz der sukzessiven Zustandsänderungen der Sensoren zu analysieren, abhängig von vorbestimmten Kriterien, welche der Vorrichtung innewohnen,
d) Mittel zur Bestimmung eines Soll-Auslöseintervalls (IEconsig), welches den geregelten Betrieb der Vorrichtung ausgehend von den analysierten Zuständen der Sensoren steuert, und
e) Mittel zur Begrenzung des Soll-Auslöseintervalls (IEconsig) auf vordefinierte Werte,
**dadurch gekennzeichnet, dass** die Begrenzungen des Soll-Auslöseintervalls (IEconsig) von dem Auslöseintervall abhängig sind, welches durch den Sensor der Aktivität (IEg) definiert wird.

2. Vorrichtung gemäß einem der vorhergehenden Ansprüche, **dadurch gekennzeichnet, dass** die Begrenzungen des Soll-Auslöseintervalls (IEconsig) dem Abzug eines vordefinierten konstanten Prozentsatzes von dem Auslöseintervall, welches durch den Sensor der Aktivität (IEg) definiert wird, entsprechen.

3. Vorrichtung gemäß einem der vorhergehenden Ansprüche, **dadurch gekennzeichnet, dass** zwei Begrenzungen angewandt werden, eine untere Grenze (IElimit_MV_Inf) und eine obere Grenze (IElimit_MV_Sup), auf das Soll-Auslöseintervall (IEconsig).

4. Vorrichtung gemäß Anspruch 3, **dadurch gekennzeichnet, dass** die zwei unteren und oberen Grenzen (IElimit_MV_Inf); (IElimit_MV_Sup) jedes Mal neu definiert werden, wenn die Mittel zur Analyse der Zustände der Sensoren bestimmen, dass das Auslöseintervall, das durch den Sensor der Aktivität (IEg) definiert ist, modifiziert wurde.

5. Vorrichtung gemäß einem der vorhergehenden Ansprüche, **dadurch gekennzeichnet, dass** die Mittel zur Begrenzung des Soll-Auslöseintervalls (IEconsig) die Begrenzung des Soll-Auslöseintervalls (IEconsig) nicht anwenden, außer in dem Fall, in dem der Sensor der Belastung (MV) in einem von der Ruhe verschiedenen Zustand ist.

6. Vorrichtung gemäß einem der vorhergehenden Ansprüche, **dadurch gekennzeichnet, dass** die Mittel zur Begrenzung des Soll-Auslöseintervalls (IEconsig) die Regelungsfrequenz begrenzen, in dem Fall, in dem der Sensor der Belastung (MV) eine Belastung erfasst, die nicht durch den Sensor der Aktivität (G) erfasst wurde.

## Claims

1. An active implantable medical device capable of functioning in an enslaved mode by driving at least one function of the device by at least one physiological parameter, said device comprising:
a) at least one effort sensor (MV) measuring a primarily physiological parameter and issuing a signal which is a function of the effort exerted by a patient bearing the device,
b) at least one activity sensor (G) measuring a primarily physical parameter,
c) means for periodically analysing the relative sequence of changes in the successive states of said sensors according to predetermined criteria intrinsic to the device,
d) means for determining a desired escape interval (IEconsig) driving the enslaved operation of the device from the analyzed states of the sensors, and
e) means for limiting the desired escape interval (IEconsig) to predetermined values,
**characterised in that** the limitations of the desired escape interval (IEconsig) are a function of the escape interval defined by the activity sensor (lEg).

2. Device according to one of the preceding claims, **characterised in that** the limitations of the desired escape interval (IEconsig) correspond to the subtraction of a predefined constant percentage of the escape interval defined by the activity sensor (IEg).

3. Device according to one of the preceding claims, **characterised in that** one applies two limitations, a lower limit (IElimit_MV_Inf) and an upper limit (IElimit_MV_Sup), to the desired escape interval (IEconsig).

4. Device according to claim 3, **characterised in that** one redefines the two lower and upper limits (IElimit_MV_Inf ; IElimit_MV_Sup) every time the means for analysing the states of the sensors determine that the escape interval defined by the activity sensor (lEg) is changed.

5. Device according to one of the preceding claims, **characterised in that** the means for limiting the desired escape interval (IEconsig) apply the limitation of the desired escape interval (IEconsig) only in the case where the effort sensor (MV) is in a state different from rest.

6. Device according to one of the preceding claims, **characterised in that** the means for limiting the desired escape interval (IEconsig) limit the enslavement frequency in the case where the effort sensor (MV) detects an effort which is not detected by the activity sensor (G).
